# DEMANDE DE BREVET EUROPEEN

(11) **EP 1 106 192 A1**
(43) Date de publication de la demande: **13.06.2001**
(21) Numéro de dépôt: 00402960.9
(22) Date de dépôt: 25.10.2000
(51) Int. Cl.: A61M 5/165

(54) **Unité de filtration d'un fluide**

(30) Priorité: 10.12.1999 FR 9915616
(71) Demandeur: MACO PHARMA, F-59200 Tourcoing (Nord) (FR)
(72) Inventeur: Verpoort, Thierry, 59420 Mouvaux (FR); Goudaliez, Francis, 59155 Faches Thumesnil (FR)
(74) Mandataire: Keib, Gérard

(57) **Abrégé**

L'invention a pour objet une unité de filtration destinée à permettre la filtration stérile d'un fluide, du type comprenant une enveloppe extérieure rigide munie d'au moins un orifice d'entrée et d'au moins un orifice de sortie, l'enveloppe renfermant un milieu filtrant, l'unité comprenant des moyens d'association étanches rapportés qui relient l'enveloppe extérieure au milieu filtrant de sorte à délimiter deux compartiments respectivement d'entrée et de sortie de l'unité de filtration.

## Description

L'invention est relative à une unité de filtration destinée à permettre la filtration stérile d'un fluide.

Elle s'applique typiquement à la filtration stérile du sang ou de composants du sang pour l'élimination des leucocytes.

On connaît déjà des unités de filtration comprenant un boîtier rigide dans lequel est disposé le milieu filtrant, notamment par compression dudit milieu entre deux parties du boîtier.

Mais de telles unités de filtration ne peuvent être stérilisées à la chaleur sèche ou humide sans risquer une déformation du boîtier rigide qui induit, par décompression locale du milieu filtrant, des passages préférentiels du fluide lors de la filtration.

En effet, par exemple lors de la stérilisation à la vapeur, généralement réalisée à 121°C, l'étanchéité entre le boîtier rigide et le milieu filtrant peut ne plus être suffisante de sorte qu'une partie du fluide passe à travers l'unité sans être filtrée.

Le document EP-A-0 526 678 décrit une unité de filtration comprenant une poche souple qui permet de résoudre ce problème.

Mais cette unité, contrairement à celle présentant un boîtier rigide, ne permet pas d'obtenir un débit de fluide filtré sensiblement constant car elle présente un volume situé en amont du milieu filtrant qui est variable.

Ce débit constant est souhaitable, notamment lorsque le débit influe sur le résultat de la filtration.

L'invention vise donc à remédier à ces inconvénients en proposant une unité de filtration qui soit stérilisable à la chaleur sèche ou humide sans risquer l'apparition de passages préférentiels et qui permet de délivrer le fluide filtré à un débit sensiblement constant.

A cet effet, l'invention propose une unité de filtration destinée à permettre la filtration stérile d'un fluide, du type comprenant une enveloppe extérieure rigide munie d'au moins un orifice d'entrée et d'au moins un orifice de sortie, l'enveloppe renfermant un milieu filtrant, l'unité comprenant des moyens d'association étanches rapportés qui relient l'enveloppe extérieure au milieu filtrant de sorte à délimiter deux compartiments respectivement d'entrée et de sortie de l'unité de filtration.

Les moyens d'association, notamment déformables et souples, sont soudés d'une part au voisinage de la périphérie extérieure du milieu filtrant et d'autre part sur la paroi interne de l'enveloppe extérieure rigide.

Selon une autre réalisation, les moyens d'association comprennent un cordon de colle disposé entre la périphérie extérieure du milieu filtrant et la paroi interne de l'enveloppe extérieure rigide.

Selon une réalisation, les moyens d'association comprennent un cadre souple dans lequel le milieu filtrant est maintenu. Par exemple, le cadre souple est formé de deux feuilles souples ajourées entre lesquelles le milieu filtrant est placé.

En variante, les feuilles formant le cadre sont fixées entre elles au niveau de la périphérie du milieu filtrant, par exemple par un cordon de soudure réalisé à travers le milieu filtrant.

Selon une réalisation, un pré filtre et/ou un post filtre sont disposés contre le milieu filtrant, respectivement du coté du compartiment d'entrée et du compartiment de sortie de l'unité de filtration.

D'autres objets et avantages de l'invention apparaîtront au cours de la description qui suit en référence aux dessins annexés.

La figure 1 représente, en vue de coté et en coupe longitudinale, un mode de réalisation de l'unité de filtration.

La figure 2 représente, en vue de face et en coupe longitudinale partielle, les moyens d'association du mode de réalisation de la figure 1, montrant en particulier l'assemblage du milieu filtrant dans un cadre souple.

Une unité de filtration 1 destinée à permettre la filtration d'un fluide comprend typiquement une enveloppe extérieure 2 munie d'au moins un orifice d'entrée 3 et d'au moins un orifice de sortie 4, l'enveloppe 2 renfermant un milieu filtrant 5.

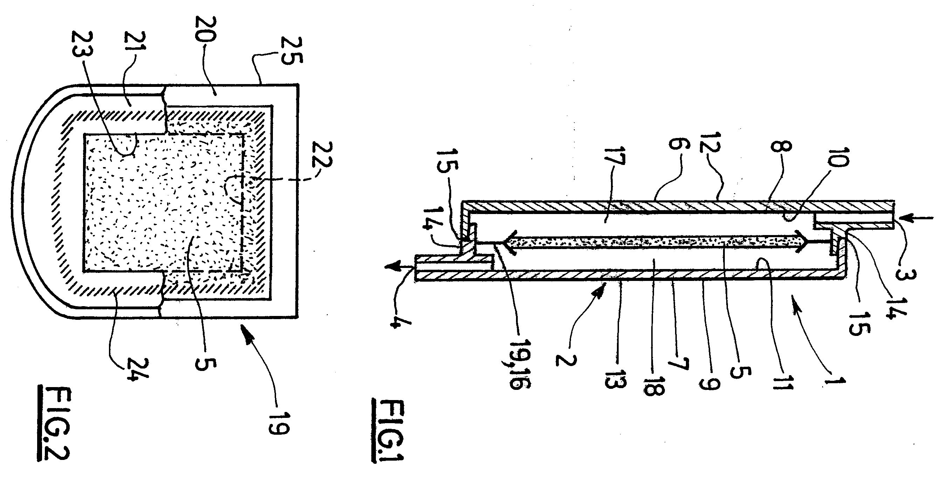

Dans la description, les termes « entrée » et « sortie » sont définis par rapport au sens de circulation du fluide dans l'unité de filtration 1 (voir les flèches représentées sur la figure 1) et les termes « interne » et « externe » en relation avec l'unité de filtration 1 assemblée.

Selon une réalisation particulière, le fluide est un fluide biologique, notamment du sang ou au moins un composant du sang, et le milieu de filtration 5 permet de retenir les leucocytes.

A cet effet, un pré filtre et/ou un post filtre peuvent être disposés contre le milieu filtrant 5 de sorte à améliorer la déplétion des leucocytes dans le fluide.

L'unité de filtration 1 est destinée à être intégrée, notamment par l'intermédiaire de tubulures connectées respectivement à l'orifice d'entrée 3 et de sortie 4, à un système comprenant par exemple des poches à usage médical, des tubulures, des clamps ou d'autres unités de filtration.

Dans de tel système, l'unité de filtration 1 est disposée sur le chemin d'écoulement du fluide de sorte que le fluide entre dans l'unité de filtration par l'orifice d'entrée 3 et que le fluide filtré soit délivré par l'intermédiaire de l'orifice de sortie 4.

Dans un exemple particulier un tel système permet la filtration du sang total puis la centrifugation du sang filtré de sorte à pouvoir le séparer en ses différents constituants. A cet effet, l'unité de filtration 1 est connectée par son entrée 3 à une poche de recueil du sang total et par sa sortie 4 à un ensemble de poches satellites. Ce système forme donc un circuit clos.

Ces différents systèmes ne sont pas décrits plus avant, dans la mesure où ils comprennent l'unité de filtration 1 selon la structure décrite ici.

Préalablement à son utilisation l'unité de filtration 1 et/ou le système qui l'intègre peut avoir subit une stérilisation à la vapeur de sorte à assurer une filtration stérile du fluide. Cette étape est notamment utilisée lorsque le système comprend une poche de recueil contenant une solution anti-coagulante ou une solution de conservation.

On décrit maintenant, en relation avec la figure 1, un mode de réalisation de l'unité de filtration 1 comprenant une enveloppe rigide 2 formée par l'assemblage de deux demi-coques 6, 7 complémentaires, par exemple réalisées en matériau plastique rigide tel que le polycarbonate.

Chaque demi-coque 6, 7 comprend une paroi latérale 8, 9 présentant une face interne 10, 11 sur l'extrémité de laquelle est formé un orifice 3, 4 s'entendant sensiblement parallèlement à la paroi 8, 9, et une face externe 12, 13.

Sur chaque demi-coque 6, 7, une première 14 et une deuxième 15 bride de fixation sont formées d'une part sur une face de l'orifice 3, 4 opposée à la paroi 8, 9 et d'autre part à l'extrémité de la face interne 10, 11 de la paroi 8, 9 opposée à l'orifice 3, 4. Par exemple, la forme de la première bride 14 est complémentaire de la forme de la deuxième bride 15.

Les deux demi-coques 6, 7 complémentaires sont disposées tête-bêche avec leurs brides de fixation 15, 15 respectives en regard, la première bride 14 de la première demi-coque 6 étant en contact avec la deuxième bride 15 de la deuxième demi-coque 7 et vice versa.

L'assemblage des deux demi-coques 6, 7 est réalisé par exemple par soudage ou collage de sorte que le volume de l'unité de filtration 1 défini entre les deux surfaces internes 10, 11 de chaque paroi 8, 9 ne communique avec l'extérieur que par l'intermédiaire des deux orifices 3, 4.

L'unité de filtration 1 comprend un milieu filtrant 5 relié à l'enveloppe extérieure 2 par l'intermédiaire de moyens d'association étanches rapportés 16 de sorte à délimiter deux compartiments respectivement d'entrée 17 et de sortie 18 de l'unité de filtration 1. Dans cette configuration, la communication fluidique entre ces deux compartiments 17, 18 est réalisée exclusivement à travers le milieu filtrant 5.

Le compartiment d'entrée 17 communique avec l'extérieur de l'unité de filtration 1 par l'intermédiaire d'une tubulure d'entrée qui sert à son remplissage avec le fluide.

Le compartiment de sortie 18 communique avec l'extérieur de l'unité de filtration 1 par l'intermédiaire d'une tubulure de sortie qui assure la délivrance du fluide filtré.

La structure de l'unité de filtration 1 permet ainsi au fluide d'être reçu dans le compartiment d'entrée 17 via l'orifice d'entrée 3, de traverser le milieu filtrant 5, puis le fluide filtré est reçu dans le compartiment de sortie 18 pour être délivré via l'orifice de sortie 4.

Selon une forme de réalisation, les tubulures d'entrée et/ou de sortie sont souples, sécables et soudables.

Dans le cas où un ensemble de poches satellites est associé à la tubulure de sortie, cette forme de réalisation permet, après dissociation de l'unité de filtration 1 par coupure et soudage de la tubulure de sortie, de pouvoir centrifuger l'ensemble de poches satellites de sorte à séparer les différents constituants du sang.

On décrit maintenant, en relation avec la figure 2, un mode de réalisation des moyens d'association étanches rapportés 16.

Par moyens rapportés, on entend notamment que les moyens d'association 16 sont réalisés en un matériau de nature différente de celui du milieu de filtration 5 et sont associés au milieu de filtration 5.

Dans un exemple particulier, les moyens d'associations 16 sont souples.

Les moyens d'association 16 représentés sur la figure 2 sont réalisés par un cadre souple 19 formé par un assemblage de deux feuilles étanches 20, 21, par exemple en PVC, entre lesquelles est placé le milieu filtrant 5.

Ces deux feuilles 20, 21 sont ajourées dans leur partie centrale et comportent chacune au moins une ouverture 22, 23 permettant le passage du fluide à filtrer.

Les deux feuilles 20, 21 sont fixées entre elles de préférence au niveau de la périphérie du milieu filtrant 5, par exemple par un cordon de soudure 24, réalisé à travers le milieu filtrant 5, assurant à la fois la fixation du milieu filtrant 5 mais également l'étanchéité entre le compartiment d'entrée 17 et le compartiment de sortie 18 de l'unité de filtration 1.

La soudure des feuilles 20, 21 à travers le milieu filtrant 5 provoque une compression, formant un cordon étanche autour du milieu filtrant 5.

L'ensemble formé par le milieu filtrant 5 et les moyens d'association 16 est relié à l'enveloppe extérieure 2 par exemple par soudage ultrason ou par collage de l'extrémité 25 des moyens d'association 16 opposée au milieu filtrant 5 sur la paroi de l'enveloppe extérieure 2.

Selon une réalisation (non représentée) les deux feuilles 20, 21 formant les moyens d'association 16 ne sont pas identiques de sorte qu'une première feuille 20, 21 est reliée à l'enveloppe extérieure 2 tandis que l'autre est seulement associée à cette première feuille.

Dans le cas où les moyens d'association 16 sont collés sur l'enveloppe extérieure 2, on peut prévoir un cordon de colle, par exemple de type silicone, sur la périphérie des moyens d'association 16 de sorte à assurer une meilleure étanchéité de la liaison.

Sur la figure 1, la liaison est réalisée sur la face interne de la paroi rigide au voisinage de la jonction entre les brides 14, 15 respectives de chaque demi-coque 6, 7.

Dans un mode de réalisation (non représenté) les moyens d'association 16 peuvent être pincés de façon étanche entre les deux demi-coques 6, 7 lors de l'association de celles-ci. En effet, les moyens d'association 16 étant étanches, aucun passage préférentiel de fluide n'est observé après stérilisation à la vapeur, contrairement au cas où le milieu filtrant 5 est directement comprimé entre deux demi-coques.

Dans un autre mode de réalisation (non représenté) les moyens d'association 16 sont réalisés par un cordon de colle, par exemple de type silicone, disposé entre la périphérie du milieu filtrant 5 et la face interne de la paroi rigide, de sorte à assurer à la fois la fixation du milieu filtrant 5 et l'étanchéité entre le compartiment d'entrée 17 et le compartiment de sortie 18 de l'unité de filtration 1.

On décrit maintenant sommairement le procédé de montage de l'unité de filtration 1 représentée sur la figure 1.

Les moyens d'association 16, préalablement fixé au milieu filtrant 5, sont associés à une première demi-coque 6, notamment par soudage ultrason ou collage.

La deuxième demi-coque 7 est ensuite disposée tête-bêche sur la première 6 avec les brides de fixation 14, 15 respectives en regard.

L'association étanche des deux demi-coques 6, 7 est ensuite réalisée par soudage ultrason ou collage.

L'unité de filtration 1 ainsi obtenue permet donc de concilier les avantages du boîtier rigide, notamment en délivrant un débit sensiblement constant lors de la filtration, tout en pouvant être stérilisé à la chaleur sèche ou humide sans induire de passages préférentiels de fluide lors de la filtration.

## Revendications

1. Unité de filtration (1) destinée à permettre la filtration stérile d'un fluide, du type comprenant une enveloppe extérieure (2) rigide munie d'au moins un orifice d'entrée (3) et d'au moins un orifice de sortie (4), l'enveloppe (2) renfermant un milieu filtrant (5), caractérisée en ce qu'elle comprend des moyens d'association étanches rapportés (16) qui relient l'enveloppe extérieure (2) au milieu filtrant (5) de sorte à délimiter deux compartiments respectivement d'entrée (17) et de sortie (18) de l'unité de filtration (1).

2. Unité de filtration selon la revendication 1, caractérisée en ce que les moyens d'association (16) sont soudés d'une part au voisinage de la périphérie extérieure (25) du milieu filtrant (5) et d'autre part sur la paroi interne de l'enveloppe extérieure (2) rigide.

3. Unité de filtration selon la revendication 1, caractérisée en ce que les moyens d'association (16) comprennent un cordon de colle disposé entre la périphérie extérieure (25) du milieu filtrant (5) et la paroi interne de l'enveloppe extérieure (2) rigide

4. Unité de filtration selon l'une quelconque des revendications 1 à 3, caractérisée en ce que les moyens d'association (16) sont déformables, notamment souples.

5. Unité de filtration selon la revendication 4, caractérisée en ce que les moyens d'association (16) comprennent un cadre souple (12) dans lequel le milieu filtrant (5) est maintenu.

6. Unité de filtration selon la revendication 5, caractérisée en ce que le cadre souple (12) est formé de deux feuilles (20, 21) souples ajourées entre lesquelles le milieu filtrant (15) est placé.

7. Unité de filtration selon la revendication 6, caractérisée en ce que les feuilles (20, 21) formant le cadre souple (12) sont fixées entre elles au niveau de la périphérie du milieu filtrant (5).

8. Unité de filtration selon la revendication 7, caractérisée en ce que la fixation des feuilles (20, 21) formant le cadre souple (12) est un cordon de soudure (24) réalisé à travers le milieu filtrant (5).

9. Unité de filtration selon l'une quelconque des revendications 1 à 8, caractérisé en ce que un pré filtre et/ou un post filtre est disposé contre le milieu filtrant (5), respectivement du coté du compartiment d'entrée (17) et du compartiment de sortie (18) de l'unité de filtration (1).

10. Unité de filtration selon l'une quelconque des revendications 1 à 9, caractérisée en ce que le milieu filtrant (5) permet la filtration du sang total ou de composants sanguins pour retenir par filtration les leucocytes.
